# EUROPÄISCHE PATENTSCHRIFT

(11) **EP 0 355 711 B1**
(45) Veröffentlichungstag und Bekanntmachung des Hinweises auf die Patenterteilung: **28.06.1995**
(21) Anmeldenummer: 89115179.7
(22) Anmeldetag: 17.08.1989
(51) Int. Cl.: C08L 23/08, A61M 5/14, A61L 29/00

(54) **Schlauch für ein Infusionsgerät**
Tubing for an infusion device
Tuyau pour dispositif de perfusion

(30) Priorität: 18.08.1988 DE 8810468 U
(43) Veröffentlichungstag der Anmeldung: 28.02.1990
(73) Patentinhaber: Ohmeda GmbH + Co. KG, 91054 Erlangen (DE); Pharmacia GmbH, 91052 Erlangen (DE)
(72) Erfinder: Iwatschenko, Peter, D-8524 Neunkirchen (DE); Hofmann, Wolfgang, D-8551 Heroldsbach (DE); Wolkenstörfer, Reinhold, D-8524 Neunkirchen (DE)
(74) Vertreter: Behrens, Dieter, Dr.-Ing.

(56) Entgegenhaltungen:
- DE-A- 2 932 982
- DE-A- 3 200 264
- FR-A- 2 376 666
- Römpps Chemie-Lexikon, 8. Auflage, Seite 1747
- K. Biederbick, Kunststoffe - kurz und bündig, 4. Auflage, Vogel- Buchverlag, Würzburg 1977, Seite 208

## Beschreibung

Die Erfindung betrifft die Verwendung eines Schlauchs für ein Infusionsgerät sowie ein Infusionsgerät.

Für die (enterale oder parenterale) Verabreichung von Flüssigkeiten mittels Infusions- oder Transfusionsgeräten haben sich für den einmaligen Gebrauch vorgesehene Packungen weitgehend durchgesetzt. Solche Packungen enthalten herkömmlicher Weise einen Schlauch aus flexiblem Material, wie PVC (Polyvinylchlorid), sowie eine sogenannte Regulierklemme, mittels welcher der Strömungsquerschnitt des Schlauches veränderbar ist, um die Fließgeschwindigkeit der Flüssigkeit nach Wunsch einstellen zu können. Typische Fließgeschwindigkeiten sind 20 bis 120 ml/h, was etwa 25 bis 60 Tropfen pro Minute entspricht. Bei bekannten Regulierklemmen werden solche Fließgeschwindigkeiten dadurch eingestellt, daS der Schlauch mittels der Regulierklenune bis auf eine Spaltbreite von etwa 4/100 mm zusammengequetscht wird.

Der Begriff "Schlauch" im Sinne der Erfindung meint die Verbindung zwischen einem eine Infusionslösung enthaltenden Behälter, wie z.B. ein Beutel, und dem Patienten (zum Beispiel über einen in den Patienten eingeführten Katheter). Die vorstehend genannten Beutel werden in der Regel aus langen "Schläuchen" durch Abtrennen einzelner Schlauchstücke hergestellt. Mit solchen "Schläuchen" hat die Erfindung aber nichts zu tun, da bei den derart hergestellten Beuteln andere Probleme als bei der vorliegenden Erfindung im Vordergrund stehen.

Auch werden bisweilen kurze Ansatzstücke mit wenigen Zentimetern Länge, die in die vorstehend genannten Beutel eingeführt sind und Anschlüssen an den Beutel dienen, häufig mit "Schlauch" bezeichnet. Auch diese "Schläuche" betrifft die vorliegende Erfindung nicht.

Regulierklemmen sind aus der DE-OS 20 43 551, DE-PS 22 42 539, DE-OS 27 41 594, US-PS 32 15 395 sowie der DE-OS 35 42 899 bekannt.

Ist die Fließgeschwindigkeit der dem Patienten zu verabreichenden Flüssigkeit einmal eingestellt, so soll sie sich möglichst nicht mehr unwillkürlich ändern. Die bekannten Schläuche für Infusionsgeräte aus PVC haben aber die Eigenschaft, sich unter Druck plastisch zu verformen. Dieser sogenannte "Kaltfluß" des Kunststoffes bewirkt langfristig eine Änderung des Strömungsquerschnittes, so daS die geforderte Konstanz der Fließgeschwindigkeit nicht immer gewährleistet ist.

Ein weiterer Nachteil des Standes der Technik ergibt sich aus der starken Temperaturabhängigkeit der physikalischen Eigenschaften des PVC, wodurch sich ebenfalls der Flußquerschnitt des Schlauches selbsttätig verändern kann.

Im Stand der Technik (s.o.) wird versucht, einer durch Kaltfluß bedingten Änderung des Strömungsquerschnittes durch eine besondere Ausgestaltung der Regulierklemmen zu begegnen.

Herkömmliche Schläuche aus PVC für Infusionsgeräte werden in der Regel mit DEHP (Diethylhexylphthalat) weichgemacht. Neuerdings wird als Weichmacher auch TOTM (Trioctyltrimellitat) eingesetzt. Die übliche Weichmacherkonzentration beträgt 30 bis 45 %. Die bekannte Technik hat allerdings den Nachteil, daß DEHP fettlöslich ist und deshalb bei Kontakt mit fetthaltigen nutritiven Substanzen aus der Schlauchwandung herausgelöst wird und somit in den Körper des Patienten gelangt. Eine Reihe von Medikamenten, wie z.B. Diazepam, Heparin oder Insulin werden von der Schlauchwandung absorbiert.

Auch haben herkömmliche Schläuche aus PVC den Nachteil, daß dann, wenn sie mit einem Ethylenoxid enthaltenden Sterilisationsmittel sterilisiert werden, eine Reaktion des Chlors des PVC mit dem Ethylenoxid zu Ethylen-Chlorhydrin stattfindet.

Aus der DE-OS 32 39 475 sind Schläuche für medizinische Geräte bekannt, die aus Ethylen-Vinylacetat (EVA) bestehen. Erkenntnisse bezüglich einer besonders vorteilhaften Zusammensetzung und insbesondere des Vinylacetatanteils finden sich nicht.

Auch die DE-OS 29 32 982 beschreibt Schläuche aus EVA. Es geht aber nicht um den oben erläuterten sogenannten Kaltfluß, sondern um andere Eigenschaften des Schlauchmaterials, nämlich dessen Sterilisationsfähigkeit, Hitzebeständigkeit, Durchsichtigkeit, niedrigen Modul und die Verschweißbarkeit. Die Druckschrift lehrt, daß die Kombination von zwei verschiedenen Ethylen/Vinylacetat-Mischpolymerisaten zum Erfolg führt. Es wird gelehrt, ein Material zu verwenden, welches verschiedene Schmelzindices für die beiden Komponenten aufweist. Das in der DE-OS 29 32 982 beschriebene Material ist für eine Schweißung geeignet. Eine mechanische Quetschverbindung ist nicht vorgesehen. Die in der DE-PS 29 32 982 beschriebenen aufwendigen Schweißverfahren sind nicht geeignet, das Schlauchmaterial mit anderen bei Bauteilen von Infusionsgeräten verwendeten Materialien, wie ABS, PP oder PE zu verbinden. Die Herstellung des Polymerisates aus zwei Komponenten mit unterschiedlichen Schmelzindices ist relativ aufwendig.

Aus der FR 78 00 365 sind Schläuche für Infusionsgeräte aus Ethylen-Vinylacetat bekannt, die aber kreuzvernetzt sind. Auch aus der DE-OS 32 00 264 sind kreuzvernetzte Ethylen-Vinylacetat-Copolymere bekannt, allerdings nicht für den Schlauch eines Infusionsgerätes, sondern für ein Schlauchansatzstück an einem sterilisierbaren Beutel.

Für Infusionspumpen mit linearer Peristaltik als Förderprinzip (US-PS 35 96 515, 37 36 930 und 40 37 598) werden derzeit sogenannte druckfeste Infusionsgeräte eingesetzt. Dabei sind eine Vielzahl von Pumpen im Gebrauch, die im Falle einer distal des Pumpenkopfes auftretenden Okklusion im Schlauch erheblich höhere Binnendrucke als 2 bar aufbauen. Im Extremfall kann es bei einer Umgebungstemperatur von 20°C zu Drucken von 10 bar kommen. Solchen Drucken halten Infusionsgeräte mit handelsüblichen PVC-Schläuchen nur bedingt stand, insbesondere dann, wenn die Temperatur des Schlauches höhere Werte erreicht. Es genügt dabei, daß die an den Patienten angeschlossene Schlauchleitung in üblicher Weise auf der Haut fixiert wird, so daß der Schlauch in diesem Abschnitt auf die Körpertemperatur des Patienten erwärmt wird. Im Falle einer unbeabsichtigten Okklusion durch Verlegung der Venenkanüle oder durch einen Knick im Schlauch selbst, fördert die Pumpe weiter. Die Festigkeit der Schlauchwandung ist nun kleiner als der maximale Pumpendruck, so daß sich der Schlauch aufbläht und platzt (ohne daS der Abschaltdruck der Pumpe erreicht wird).

Ein weiteres wichtiges Kriterium für die Qualität von Infusions-Apparaten ist die sogenannte Alarmverzögerungszeit. Damit ist die Zeitspanne gemeint, die zwischen dem Eintreten eines Störfalles, beispielsweise einer Okklusion, und dem Abschalten und der Alarmgabe der Vorrichtung verstreicht. Diese Zeitspanne muß so kurz wie möglich gehalten werden, da der Patient bei einem Störfall u.U. lebensnotwendige Medikamente nicht mehr erhält. Bei kleinen Infusionsgeschwindigkeiten von z.B. 3 Tropfen pro Minute oder 10 ml/h haben Messungen zur Alarmverzögerungszeit ergeben, daß diese beim Stand der Technik mehrere Stunden betragen kann.

Auch die Sterilisation von Infusionsgeräten und insbesondere deren Schläuchen wirft besondere Probleme auf. In der DE-OS 29 32 982 wird darauf hingewiesen, daß die dort vorgeschlagene EVA-Mischung besonders vorteilhafte Strahlensterilisationseigenschaften aufweise. Das in der DE-OS 32 39 475 beschriebene thermische Sterilisationsverfahren stellt besondere Ansprüche an die Verbindungen zwischen den einzelnen Bauteilen, also insbesondere die Befestigung des Schlauches am Beutel etc. Die Verbindung muß durch Wärmekontakt oder Hochfrequenzschweissung hergestellt werden, da bei Sterilisieren mit Strahlen im EVA-Copolymer Temperaturen bis zu 60°C auftreten können. Beim Autoklavieren muß mit Temperaturen bis zu 120°C gearbeitet werden, um einen hinreichenden sterilisationserfolg zu erreichen.

Untersuchungen haben ergeben, daß bei mechanischen Verbindungen (z.B. Klemmung) oder bei einer einfachen Aufschiebetechnik gemäß der DE-OS 29 32 982 bei solchen Temperaturen, bereits eine plastische Verformung des Materials eintritt. Derartige EVA-Copolymere haben sich deshalb als für solche Sterilisationsverfahren ungeeignet erwiesen, da ihr Schmelzpunkt zwischen 70°C und 90°C liegt. Der Schlauch würde durch die plastische Verformung seine Eigenspannung verlieren.

Der Erfindung liegt die Aufgabe zugrunde, die vorstehend beschriebenen Nachteile zu überwinden und ein mit einer Regulierklemme versehenes Infusionsgerät mit einem Schlauch zu schaffen, der eine konstante Fließgeschwindigkeit ermöglicht, gegen eine Wechselwirkung mit den zu verabreichenden Substanzen weitestgehend inert ist, eine mechanische Befestigung an anderen Bauteilen (Beutel etc.) ermöglicht, eine einfache, kostengünstige und wirksame Sterilisation erlaubt, eine einfache und kostengünstige Herstellung des Infusionsgerätes ermöglicht, und gute mechanische Eigenschaften, insbesondere eine gute Knickfestigkeit, aufweist.

Erfindungsgemäß wird die Verwendung eines Schlauchs vorgeschlagen, der im wesentlichen aus einem unvernetzten, homogenen Ethylen-Vinylacetat-Copolymer mit einem Vinylacetat-Anteil von 12 bis 28 % besteht und 0 bis 25 Gew.-% eines Zusatzes von Thermoplasten und/oder Elastomeren, ausgenommen Ethylen-Vinylacetat-Copolymere, enthält, in einem Infusionsgerät zur enteralen oder parenteralen Verabreichung von nutritiven oder medikamentösen Flüssigkeiten als Verbindung von einem die Flüssigkeit enthaltenden Behälter zum Patienten, wobei die Verbindung mit einer Regulierklemme zur Veränderung des Strömungsquerschnitts der Verbindung versehen ist.

Erfindungsgemäß wird ferner ein Infusionsgerät vorgeschlagen mit einem Schlauch (12) für die enterale oder parenterale Verabreichung von nutritiven oder medikamentösen Flüssigkeiten und einer Regulierklemme (10), die den Schlauch (12) zur Einstellung einer Durchflußmenge der Flüssigkeiten zusammendrückt, dadurch gekennzeichnet, daß der Schlauch (12) im wesentlichen aus einem unvernetzten, homogenen Ethylen-Vinylacetat-Copolymer mit einem Vinvlacetat-Anteil von 12 bis 28 % besteht und 0 bis 25 Gew.-% eines Zusatzes von Thermoplasten und/oder Elastomeren, ausgenommen Ethylen-Vinylacetat-Copolymere, enthält.

Der vorstehende Begriff "im wesentlichen" besagt, daß Ethylen-Vinylacetat als Hauptbestandteil des Schlauches vorgesehen ist und Zusätze im Bereich von einigen Prozent nicht ausgeschlossen sind.

Der Erfindung liegt also die Erkenntnis zugrunde, daß im Unterschied zum Stand der Technik keine inhomogene EVA-Mischung, sondern ein homogenes Copolymer von Vorteil ist. Das Ethylen und das Vinylacetat sind zusammen polymerisiert. Weiter liegt der Erfindung die Erkenntnis zugrunde, daß ganz bestimmte Grenzwerte bezüglich des Vinylacetatanteils eingehalten werden müssen, um günstige Ergebnisse zu erzielen.

Der Erfindung liegt weiter die Erkenntnis zugrunde, daß (im Unterschied zur obengenannten FR 78 00 365 und zur DE 32 00 264) eine Kreuzvernetzung des Ethylen-Vinylacetat-Copolymers nicht erforderlich ist. Es wurde gefunden, daß eine solche Kreuzvernetzung des Schlauchmaterials Nachteile beim Zusammenwirken mit der Regulierklemme hätte. Das Kaltfließverhalten würde bei einer solchen Kreuzvernetzung deutlich verschlechtert. Die Erfindung verwendet deshalb bevorzugt als Schlauchmaterial homogenes Ethylen-Vinylacetat-Copolymer mit den in den Ansprüchen angegebenen Vinylacetatanteilen bzw. Zusätzen, das nicht kreuzvernetzt ist.

In einer bevorzugten Ausgestaltung beträgt der Vinylacetatanteil 12 bis 25 %.

Untersuchungen haben gezeigt, daß erfindungsgemäß verwendete EVA-Schläuche, deren Vinylacetat-Anteil zwischen 18 und 20 % liegt, hervorragende physikalische und chemische Eigenschaften in bezug auf die oben beschriebenen Kriterien aufweisen. Mit solchen Schläuchen wird ein sicheres Abschalten des Apparates unter gleichzeitiger Alarmgabe gewährleistet, das Speichervolumen wird minimiert und die Alarmverzögerungszeit beträgt auch bei kleinsten Infusionsraten nur noch wenige Minuten.

Erfindungsgemäß verwendete Schläuche können in einfacher Weise durch mechanische Klemmung mit anderen Bauteilen verbunden werden, ohne daß Probleme bei der Sterilisation auftreten.

Ethylen-Vinylacetat (EVA) ist ein weichmacherfreier Werkstoff aus der Gruppe der Elastomere. Bei einem Vinylacetat-Anteil (Vinylacetat = VA) von 12 bis 28 %, eignet sich dieses Material besonders gut zur Herstellung von Schläuchen von Infusionsgeräten. EVA zeichnet sich, insbesondere mit dem angegebenen VA-Anteil, durch sein inertes Verhalten gegenüber nutritiven und medikamentösen Substanzen aus. Wird ein Infusionsgerät mit einer Regulierklemme, insbesondere einer Rollenklemme, verwendet, so wird eine konstante Flußgeschwindigkeit auch über lange Zeiträume erzielt, weil das Material in weiten Temperaturbereichen ein konstantes elastisches Verhalten aufweist, so daß die mit der Regulierklemme einmal eingestellte Flußgeschwindigkeit beibehalten wird. Der bei PVC zu beklagende Kaltfluß tritt nicht in merklichem Umfang auf.

Ein weiterer Vorteil der Verwendung von EVA als Material für den Schlauch des Infusionsgerätes ergibt sich bei der Herstellung. Wird der Schlauch nämlich mit Ethylenoxid sterilisiert, so zeigt sich, daß das Desorptionsverhalten von Ethylenoxid bei EVA etwa um den Faktor 10 günstiger ist als bei einem Schlauch aus PVC. Der maximal zulässige Rest an Ethylenoxid soll bei Schläuchen für Infusionsgeräte geringer sein als 1 ppm. Nach der Sterilisation mit Ethylenoxid muß also das Material solange einer Anwendung ferngehalten werden, bis aufgrund der Desorption das Ethylenoxid bis auf den zugelassenen Rest desorbiert ist. Somit können aus EVA gefertigte Infusionsgeräte und Schläuche wesentlich schneller nach der Sterilisation in den Verkehr gebracht werden als Geräte aus PVC. Kostenaufwendige Verfahren zur Beschleunigung der Desorption erübrigen sich.

Den vorstehend beschriebenen erfindungsgemäß verwendeten Schläuchen aus EVA können gemäß bevorzugten Ausgestaltungen der Erfindung Zusätze aus Thermoplasten und/oder Elastomeren hinzugefügt werden, wobei aber als Hauptbestandteil EVA vorgesehen ist. So haben sich Zusätze aus Polyurethan und/oder ABS (Acrylnitril-Butadien-Styrolcopolymerisat) und/oder thermoplastischem Polyester als vorteilhaft hinsichtlich der Verklebbarkeit des Materials mit anderen Werkstoffen erwiesen. Der Zusatz kann bis zu 25 % betragen, vorzugsweise bis zu 10 %. Die hier verwendeten %-Angaben beziehen sich auf Gew.-%.

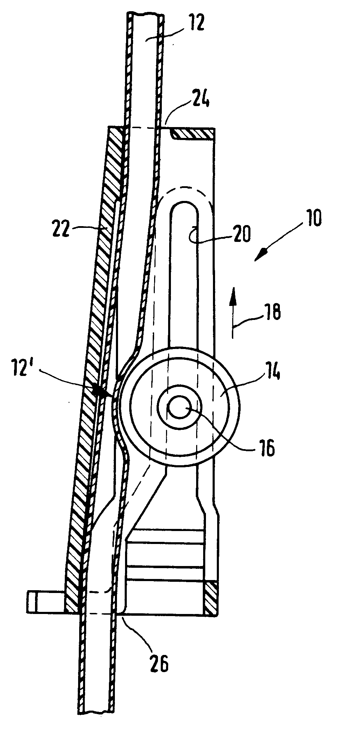

Nachfolgend wird ein Ausführungsbeispiel der Erfindung anhand der Zeichnung näher beschrieben.

Die Figur zeigt einen wesentlichen Teil eines Infusionsgerätes einschließlich eines Schlauches und einer Rollenklemme.

Eine als solche bekannte Rollenklemme 10 beaufschlagt einen Schlauch 12, durch den eine nutritive oder medikameutöse Substanz in einen Patienten eingebracht wird. Die übrigen, als solche bekannten Teile des Infusionsgerätes, wie insbesondere eine Tropfkammer und eine Kanüle, sind nicht gezeigt und brauchen hier auch nicht beschrieben zu werden.

Der Schlauch 12 weist an der Stelle 12' eine Verengung auf, durch welche die Strömungsgeschwindigkeit der Flüssigkeit durch den Schlauch definiert wird. Die Verengung 12' des Schlauches 12 wird mittels einer Rolle 14 der Rollenklemme eingestellt. Die Rolle 14 weist in bekannter Weise eine Welle 16 auf, die auf beiden Seiten derart geführt ist, daß die Rolle in Richtung des Pfeiles 18 zum Verändern des Strömungsquerschnittes des Schlauches 12 verschiebbar ist. Die Rolle 14 und ihre Welle 16 werden also in einem Kanal 20 in Richtung des Pfeiles 18 verschoben, wobei sich der Abstand zwischen dem Umfang der Rolle 14 und einer Rampe 22 der Rollenklemme verringert, so daß in Abhängigkeit von der Position der Rolle 14 der Strömungsquerschnitt des Schlauches 12 veränderbar ist. Der Schlauch tritt bei 24 in die Rollenklemme 10 ein und bei 26 aus ihr aus.

Der Schlauch besteht aus Ethylen-Vinylacetat-Copolymer mit einem Vinylacetat-Anteil von 12 bis 28 %. Er ist mit Ethylenoxid sterilisiert worden.

## Patentansprüche

1. Verwendung eines Schlauchs, der im wesentlichen aus einem unvernetzten, homogenen Ethylen-Vinylacetat-Copolymer mit einem Vinylacetat-Anteil von 12 bis 28 % besteht und 0 bis 25 Gew.-% eines Zusatzes von Thermoplasten und/oder Elastomeren, ausgenommen Ethylen-Vinylacetat-Copolymere, enthält, in einem Infusionsgerät zur enteralen oder parenteralen Verabreichung von nutritiven oder medikamentösen Flüssigkeiten als Verbindung von einem die Flüssigkeit enthaltenden Behälter zum Patienten, wobei die Verbindung mit einer Regulierklemme zur Veränderung des Strömungsquerschnitts der Verbindung versehen ist.

2. Verwendung eines Schlauchs nach Anspruch 1, dadurch gekennzeichnet, daß der Vinylacetat-Anteil 12 bis 25% beträgt.

3. Verwendung eines Schlauchs nach Anspruch 1, dadurch gekennzeichnet, daß der Vinylacetat-Anteil geringer als 20% ist.

4. Verwendung eines Schlauchs nach Anspruch 1, dadurch gekennzeichnet, daß der Vinylacetat-Anteil zwischen 18 und 20% liegt.

5. Verwendung eines Schlauchs nach einem der vorhergehenden Ansprüche, dadurch gekennzeichnet, daß er mit Ethylenoxid sterilisiert ist.

6. Verwendung eines Schlauchs nach Anspruch 1, dadurch gekennzeichnet, daß er einen Polyurethan-Zusatz enthält.

7. Verwendung eines Schlauchs nach Anspruch 1, dadurch gekennzeichnet, daß er einen ABS-Zusatz enthält.

8. Verwendung eines Schlauchs nach Anspruch 1, dadurch gekennzeichnet, daß er einen thermoplastischen Polyester-Zusatz enthält.

9. Infusionsgerät mit einem Schlauch (12) für die enterale oder parenterale Verabreichung von nutritiven oder medikamentösen Flüssigkeiten und einer Regulierklemme (10), die den Schlauch (12) zur Einstellung einer Durchflußmenge der Flüssigkeiten zusammendrückt, dadurch gekennzeichnet, daß der Schlauch (12) im wesentlichen aus einem unvernetzten, homogenen Ethylen-Vinylacetat-Copolymer mit einem Vinylacetat-Anteil von 12 bis 28 % besteht und 0 bis 25 Gew.-% eines Zusatzes von Thermoplasten und/oder Elastomeren, ausgenommen Ethylen-Vinylacetat-Copolymere, enthält.

## Claims

1. Use of a hose which consists substantially of a non cross-linked, homogenous ethylene vinyl acetate copolymer with a vinyl acetate component of 12 to 28% and which contains 0 to 25% by weight of an addition of thermoplastics and/or elastomers, excluding ethylene vinyl acetate copolymers, in an infusion apparatus for the enteric or parenteric administration of nutrative or medicative liquids as a connection from a container containing the liquid to the patient, with the connection being provided with a regulating clip to change the flow cross-section of the connection.

2. Use of a hose in accordance with claim 1, characterised in that the vinyl acetate component amounts to 12 to 25%.

3. Use of a hose in accordance with claim 1, characterised in that the vinyl acetate component is smaller than 20%.

4. Use of a hose in accordance with claim 1, characterised in that the vinyl acetate component lies between 18 and 20%.

5. Use of a hose in accordance with one of the preceding claims, characterised in that it is sterilised with ethylene oxide.

6. Use of a hose in accordance with claim 1, characterised in that it contains a polyurethane addition.

7. Use of a hose in accordance with claim 1, characterised in that it contains an ABS addition.

8. Use of a hose in accordance with claim 1, characterised in that it contains a thermoplastic polyester addition.

9. Infusion apparatus with a hose (12) for the enteric or parenteric administration of nutrative or medicative liquids and a regulating clip (10) which compresses the hose (12) for the adjustment of a quantity of the liquids flowing through it, characterised in that the hose (12) consists substantially of a non cross-linked, homogenous ethylene vinyl acetate copolymer with a vinyl acetate component of 12 to 28% and containing 0 to 25% by weight of an addition of thermoplastics and/or elastomers, excluding ethylene vinyl acetate copolymers.

## Revendications

1. Utilisation d'un tuyau, qui est constitué essentiellement d'un copolymère d'ethylène-acétate de vinyle non-réticulé et homogène avec une ProPortion de 12 à 28% d'acétate de vinyle, et contenant de 0 à 25% en poids d'un additif de thermoplastes et/ou d'élastomères, à l'exclusion de copolymères d'éthylène-acétate de vinyle, dans un appareil à infusion pour l'administration entérale ou parentérale de liquides nutritifs ou medicamenteux, en tant que liaison entre un récipient qui contient le liquide et un patient, la liaison étant pourvue d'une pince de régulation pour modifier la section d'écoulement de la liaison.

2. Utilisation d'un tuyau selon la revendication 1, caractérisée en ce que la proportion d'acétate de vinyle s'élève de 12 à 25%.

3. Utilisation d'un tuyau selon la revendication 1, caractérisée en ce que la proportion d'acétate de vinyle est inférieure à 20%.

4. Utilisation d'un tuyau selon la revendication 1, caractérisée en ce que la proportion d'acétate de vinyle est située entre 18 à 20%.

5. Utilisation d'un tuyau selon la revendications précédentes, caractérisée en ce qu'il est stérilisé à l'oxyde d'éthylène.

6. Utilisation d'un tuyau selon la revendication 1, caractérisée en ce qu'il contient un additif de polyuréthane.

7. Utilisation d'un tuyau selon la revendication 1, caractérisée en ce qu'il contient un additif d'ABS.

8. Utilisation d'un tuyau selon la revendication 1, caractérisée en ce qu'il contient un additif de polyester thermoplastique.

9. Appareil à infusion comprenant un tuyau (12) pour l'administration entérale ou parentérale de liquides nutritifs ou médicamenteux, et une pince de régulation (10) qui comprime le tuyau (12) pour établir un débit d'ecoulement du liquide, caractérisé en ce que le tuyau (12) est constitué essentiellement d'un copolymère d'éthylène-acétate de vinyle non-réticulé et homogène avec une proportion d'acétate de vinyle de 12 à 28%, et contenant de 0 à 25% en poids d'un additif de thermoplastes et/ou d'élastomères, à l'exclusion des copolymères d'éthylène-acétate de vinyle.
